# EUROPÄISCHE PATENTSCHRIFT

(11) **EP 0 687 421 B1**
(45) Veröffentlichungstag und Bekanntmachung des Hinweises auf die Patenterteilung: **06.10.1999**
(21) Anmeldenummer: 95101570.0
(22) Anmeldetag: 07.02.1995
(51) Int. Cl.: A23L 1/015, A61L 2/02, F04B 9/00

(54) **Vorrichtung zur Erzeugung eines zur Sterilisation herangezogenen Hochdruckes**
Apparatus for generating high-pressure for the purpose of sterilization
Dispositif pour la production de haute pression pour stériliser

(30) Priorität: 17.06.1994 DE 4421341
(43) Veröffentlichungstag der Anmeldung: 20.12.1995
(73) Patentinhaber: Krupp Uhde GmbH, 44141 Dortmund (DE)
(72) Erfinder: Hiltawsky, Josef, D-58239 Schwerte (DE); Körner, Jörg-Peter, D-58099 Hagen (DE); Dierkes, Heribert, D-58093 Hagen (DE); Kurtz, Hans-Ottomar, D-44139 Dortmund (DE)
(74) Vertreter: Patentanwälte Meinke, Dabringhaus und Partner

(56) Entgegenhaltungen:
- EP-A- 0 480 422
- DE-A- 3 834 996
- FR-A- 2 690 854

## Beschreibung

Die Erfindung richtet sich auf eine Vorrichtung der im Oberbegriff des Anspruches 1 angegebenen Gattung. Eine derartige Vorrichtung ist beispielsweise aus der EP-A-0 480 422 bekannt.

Um die Jahrhundertwende wurde von den damaligen Forschern auf dem Gebiet der Hochdruckanwendungen die Entdeckung gemacht, daß isostatische Drücke im Bereich von 500 bis 10.000 bar bei Raumtemperatur Mikroorganismen abtöten.

In der Zeitschrift "FOOD MANUFACTURE", November 1992, berichtet Bart Mertens in seinem Beitrag mit dem Titel "Under pressure", daß in neuerer Zeit diese Entdeckung der bei Raumtemperatur sterilisierenden Wirkung des hohen Drucks erneut aufgegriffen wurde und in der Nahrungsmittelindustrie genutzt wird.

Im wesentlichen werden hochdrucksterilisierte Früchtejoghurts, Gelees, Salatsaucen und Obstsaucen hergestellt, aber auch sterilisierte Zitrussäfte.

Die Nahrungsmittel werden in der Verkaufspackung versiegelt der isostatischen Hochdrucksterilisation unterworfen. Vornehmlich dafür geeignete Verpackungswerkstoffe sind elastoplastische Kunststoffe und Aluminiumfolie bzw. daraus hergestellte Kombinationshalbzeuge. Die daraus hergestellten Verpackungen verformen sich unter dem Druck flexibel oder halbsteif und folgen der Kompaktierung von Inhalt und nicht befülltem Restraum in der Packung. Beispielsweise nimmt das Ursprungsvolumen einer befüllten Packung um etwa 12 % ab, wenn sie mit einem allseitigen hydrostatischen Druck von 4.000 bar hochdrucksterilisiert wird. Sie wird dabei nicht beschädigt.

Hochdrucksterilisations-Autoklaven für den Einsatz in der Nahrungsmittelindustrie müssen unter wirtschaftlichen Gesichtspunkten gesehen innerhalb möglichst kurzer Zykluszeiten mit zu behandelndem Gut beschickt und davon entleert werden können. Der hydrostatische Druck muß kurzfristig im Autoklaven aufgebaut und auf dem eingestellten Niveau gehalten werden, obgleich noch in der Haltezeit die Kompaktierung der Packung bis zum Ende abläuft.

In der eingangs schon genannten EP-A-0 480 422 wird eine Sterilisationsmethode zur Konservierung von Fruchtsaft mittels Hochdruck beschrieben. Dabei wird der zu sterilisierende Fruchtsaft entweder unverpackt oder in einer versiegelten Verpackung mit dem Hochdruck beaufschlagt. Die Behandlungsdrücke reichen von 2.000 bar bis 10.000 bar, die Behandlungsdauer liegt zwischen 5 bis 120 Minuten. Behandlungsdruck und Behandlungsdauer sind produktspezifisch optimiert.

Der unverpackte Fruchtsaft wird unmittelbar in die Hochdruckkammer gefüllt. Ein Ende der Hochdruckkammer ist mit einem beweglichen Kolben verschlossen, das andere Ende mit einem Deckel. Der bewegliche Kolben wird von einem Hydraulikzylinder angetrieben.

Zur Hochdruckbehandlung des in der Kammer befindlichen Fruchtsaftes bewegt der mit Hydraulikflüssigkeit beaufschlagte Hydraulikzylinder den Kolben in Richtung auf den Deckel.

Zu jedem Zeitpunkt der Kolbenbewegung besteht ein Kräftegleichgewicht, das vom Behandlungsdruck auf der Fläche des beweglichen Kolbens einerseits und andererseits vom Hydraulikdruck auf der Fläche des Hydraulikzylinderkolbens aufgebaut wird. Wird der Hydraulikdruck erhöht, bewegt sich der Kolben in die Hochdruckkammer hinein und erhöht darin den auf dem Fruchtsaft lastenden Druck.

Die Fläche des Hydraulikzylinderkolbens ist um ein Vielfaches größer als die Fläche des Kolbens in der Hochdruckkammer. Daher kann mittels vergleichsweise niedrigem Hydraulikdruck ein um das Flächenvielfache höherer Behandlungsdruck in der Hochdruckkammer bewirkt werden. Eine solche Anordnung wird in der Hochdrucktechnik als Druckübersetzer bezeichnet.

Wenn in der Hochdruckkammer des Druckübersetzers der Inhalt von versiegelten Verpackungen sterilisiert wird, sind die Verpackungen von Transmitterflüssigkeit allseitig umgeben.

Die Transmitterflüssigkeit füllt die Hochdruckkammer voll aus. In der Transmitterflüssigkeit wird der Behandlungsdruck in der Hochdruckkammer durch die Kolbenbewegung erzeugt. Der Transmitterflüssigkeitsdruck wirkt allseitig auf die versiegelten Verpackungen in der Hochdruckkammer.

T. Makita weist in seiner Veröffentlichung "Application of high pressure and thermophysical properties of water to biotechnology" in der Zeitschrift "Fluid Phase Equlibria", Jahrgang 76, (1992), Seite 87-95, darauf hin, daß Wasser bei 20°C unter einem Druck von 8.840 bar in den festen Zustand (Eis-VI) übergeht. Wird nun Wasser als Transmitterflüssigkeit genutzt, so kann in den engen Querschnitten von hochdruckführenden Rohrleitungen eine irreversible Eis-VI-Bildung auftreten, wenn die Hochdruckbehandlungskammer und die Druckerzeugungseinheit räumlich voneinander getrennt und mittels Rohrleitung verbunden sind. Die Eis-VI-Bildung wirkt sich äußerst störend auf einen Betriebsablauf aus, bei dem eine Hochdruckkammer in zyklisch verkettete Belade-, Behandlungs- und Entladevorgänge eingebunden ist.

In dem Fachbuch "HIGH PRESSURE TECHNOLOGY, VOLUME I, Equipment Design, Materials and Properties", herausgegeben von Ian L. Spain und Jac Paauwe, 1977, Verlag MARCEL DEKKER, Inc. New York, USA, sind auf den Seiten 162 bis 167 Druckübersetzer beschrieben und in Schritten dargestellt. Bei diesen Druckübersetzern wird die auf den Deckel der Hochdruckkammer wirkende Druckkraft von der zylindrischen Wand der Hochdruckkammer aufgenommen. In der zylindrischen Wand entsteht dadurch auch eine Axialspannung.

Diese Axialspannung bewirkt im Wandwerkstoff einen dreiachsigen Spannungszustand. Die dreiachsig belastete zylindrische Wand der Hochdruckkammer muß daher vergleichsweise dicker ausgeführt werden als eine Wand, die mit einem zweiachsigen axialspannungslosen Spannungszustand belastet ist. Die dickere Wand ist kostenmäßig ungünstiger.

Die von Spain/Paauwe mitgeteilten Druckübersetzer besitzen eine eingeschränkte Zugänglichkeit zur Hochdruckkammer. Der volle Querschnitt der Hochdruckkammer wird nach Entfernen des Deckels zugänglich. Der Deckel ist jedoch kraft- bzw. formschlüssig mit der zylindrischen Wand der Hochdruckkammer verbunden, damit die Wand die Deckelkraft aufnehmen kann.

Um den Deckel zu entfernen, sind diese Verbindungen zuvor zu lösen, also entweder sind Flanschverschraubungen zu lösen oder der Deckel ist als Ganzes - als Gewindestopfen - aus der Öffnung herauszudrehen. Die zyklische Beschickung einer Hochdruckkammer, die mit einem derartigen Axialspannung erzeugenden Deckel ausgestattet ist und worin versiegelte Verpackungen behandelt werden, ist innerhalb wirtschaftlich sinnvoller Zykluszeiten von vornherein nicht zu erwarten.

Zum Problem Druckübersetzer sei noch auf die DE-C-30 32 430 verwiesen mit weiteren Literaturnachweisen. Eine Schmiedepresse, bei der die Aufhängung des Obergesenks und die Auflage des Untergesenks in einem stationären Rahmen vorgesehen sind, ist der DE-C-39 33 076 zu entnehmen.

Aufgabe der Erfindung ist die Schaffung einer Lösung, mit der eine einfache, ggf. vollautomatische Handhabung der Sterilisation möglich gemacht wird, ohne die Vorteile der bekannten Lösungen aufgeben zu müssen.

Mit einer Vorrichtung der gattungsgemäßen Art wird diese Aufgabe gemäß der Erfindung dadurch gelöst, daß die beiden Druckkammern von einem verfahrbaren Jochrahmen derart umschlossen sind, daß der Deckel der Hochdruckkammer und der Boden der Niederdruckkammer sich bei aktiviertem Arbeitsdruck gegen Innenflächen des Jochrahmens abstützen.

Mit der Erfindung wird erreicht, daß die Druckkammer konstruktiv vergleichsweise einfach gestaltet werden kann, daß das Öffnen und Schließen der Druckkammer rasch und problemlos möglich gemacht wird und daß damit kurze Arbeitszyklen erreichbar sind.

Vorteilhafte Ausgestaltungen der Erfindung ergeben sich aus den Unteransprüchen.

Dabei ist es besonders zweckmäßig, die Vorrichtung in einem Vorrichtungsrahmen federnd zu lagern, womit etwa auftretende geringfügige Maßänderungen bei Druckbelastung bzw. Druckentlastung unmittelbar über kurze Federwege abgefangen werden können.

Der Jochrahmen ist zweckmäßig auf Fahrschienen gelagert. Auch dies dient dazu, eine halbe oder vollautomatische Verfahrbarkeit möglich zu machen, etwa in der Weise, daß der Jochrahmen über Hydraulikzylinder auf den Fahrschienen verschiebbar ist.

Eine besonders zweckmäßige Ausgestaltung der Erfindung besteht darin, daß die Zylinderwand der Hochdruckkammer mehrlagig ausgebildet ist mit einem Innenmantel und einer Mehrzahl von diesem Innenmantel umgebenden bandarmierten Ringscheiben.

Derartige bandarmierte Ringscheiben sind für sich gesehen bekannt, beispielsweise aus der DE-A-38 34 996. Die bekannten Ringscheiben werden als Formmatritzen für Gesenke eingesetzt. Die Erfindung greift diese Technologie auf und benutzt sie als Gestaltung der Wände der Hochdruckbehälter.

Die Erfindung ist nachstehend anhand der Zeichnung beispielsweise näher erläutert. Diese zeigt in
- Fig. 1: eine Seitenansicht der Vorrichtung mit den Zylindern, teilweise im Schnitt im geschlossenen Zustand,
- Fig. 2: eine Ansicht gemäß Pfeil II in Fig. 1 auf die Vorrichtung wiederum teilweise im Schnitt sowie in
- Fig. 3: eine Aufsicht gemäß Pfeil III in Fig. 1 auf die Vorrichtung.

Die allgemein mit 1 bezeichnete Vorrichtung weist zunächst eine Niederdruckkammer 2 auf, die an einem allgemein mit 3 bezeichneten Vorrichtungsrahmen befestigt ist. Dieser Vorrichtungsrahmen 3 wird von Tragplatten 4 und 5 gebildet, die auf Quertraversen 6 angeordnet sind, die wiederum ihrerseits über Federelemente 7 an einem Grundrahmen 8 befestigt sind.

Der Niederdruckkammer 2 ist ein Niederdruckkolben 9 an einer Tandemkolbenstange 10 zugeordnet, die am anderen Ende einen Hochdruckkolben 11 aufweist, der eine Hochdruckkammer 12 beaufschlagt.

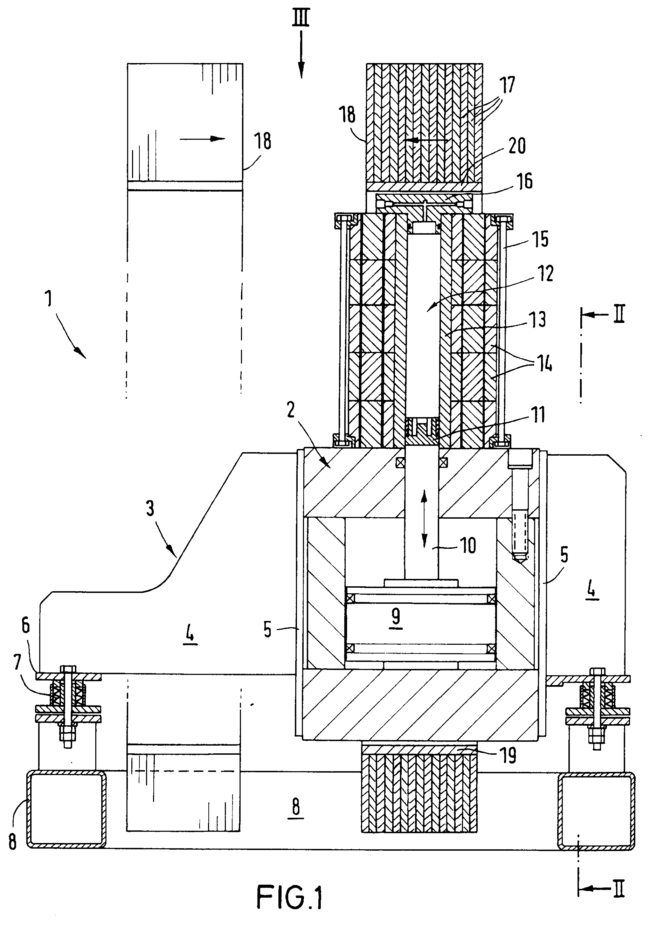

Diese Hochdruckkammer 12 ist, wie sich insbesondere aus Fig. 1 ergibt, mehrlagig ausgestaltet, sie weist einen inneren, in der Regel lebensmitteltauglichen Zylindermantel 13 auf, der von einer Mehrzahl von Ringscheiben 14 umgeben ist, wobei das Ringscheibenpaket über Zuganker 15 zusammengespannt ist.

Das in Schwerkraftrichtung obere Ende der Hochdruckkammer 12 ist von einem gedichteten Deckel 16 abgeschlossen, der über eine nicht näher dargestellte automatische Handhabungseinrichtung aufgenommen und abgesetzt werden kann.

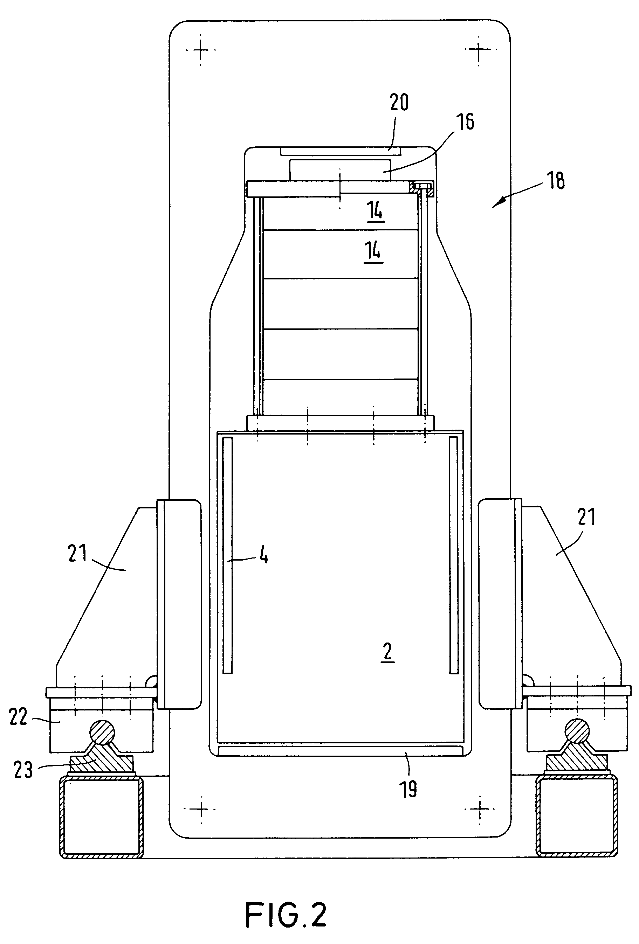

Wie sich aus den Fig. 1 und 2 ergibt, wird die Niederdruckkammer 2 und die Hochdruckkammer 12 von einem aus einer Vielzahl von Platten 17 bestehenden, verfahrbaren Jochrahmen 18 umgeben, derart, daß sich eine am Jochrahmen 18 befindliche untere Bodendruckplatte 19 an den Boden der Niederdruckkammer 12 und eine obere Deckelplatte 20 am Deckel 16 anlegen kann, wenn die Innendruckräume druckbeaufschlagt werden und das Bestreben haben, sich auszuweiten.

Durch den Jochrahmen bzw. die Jochplatten 17 in Verbindung mit der Bodenplatte 19 und der Deckelplatte 20 werden alle Elemente gegeneinander verspannt, so daß Axialkräfte nicht mehr von den Wänden der Druckkammern aufzunehmen sind, sondern im wesentlichen von den Jochplatten 17.

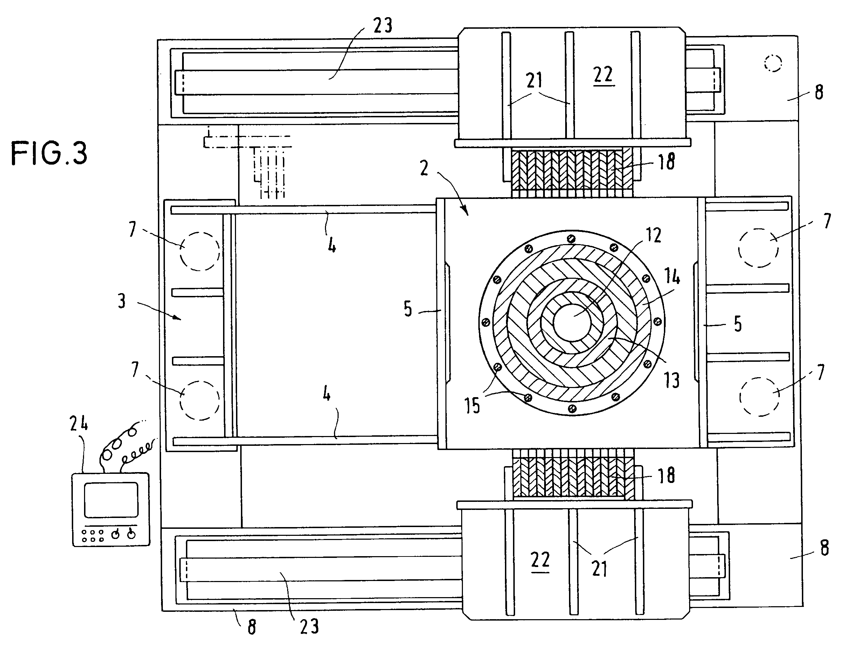

Wie sich insbesondere aus den Fig. 2 und 3 ergibt, ist der Jochrahmen 18 über seitliche Tragstützen 21 und entsprechende Gleitsteine 22 auf Gleitschienen 23 verfahrbar an der Vorrichtung 1 angeordnet. Die Verfahrbarkeit ist in Fig. 1, obere Figurenhälfte, durch kleine Pfeile angedeutet.

In Fig. 3 ist noch eine speicherprogrammierbare Steuerung 24 angedeutet, mit der Arbeitsabläufe, insbesondere auch die Verfahrbarkeit des Jochrahmens und das Auflegen bzw. Abgeben des Deckels 16, automatisierbar sind.

Natürlich ist das beschriebene Ausführungsbeispiel der Erfindung noch in vielfacher Hinsicht abzuändern, ohne den Schutzumfang der Ansprüche zu verlassen. So ist es möglich, eine Mehrzahl von taktweise arbeitenden Vorrichtungen mit durchlaufenden Jochelementen in einer Schlange vorzusehen, um hohe Durchsetzleistungen zu erreichen bei einer Mehrzahl von Hoch- und Niederkammern in Folge u. dgl. mehr.

## Patentansprüche

1. Vorrichtung zur Erzeugung eines zur Sterilisation herangezogenen Hochdruckes mit einer ersten Hochdruckkammer als Arbeitskammer und einer zweiten Niederdruckkammer größeren Durchmessers, wobei die beiden Druckkammern von einem Tandemkolben mit unterschiedlich großen Kolbenflächen als Druckübersetzer beaufschlagt sind und der Tandemkolben auf seiner großen Fläche durch extern erzeugten Hydraulikdruck aktivierbar ist und dadurch mit seiner kleinen Fläche den Arbeitsdruck in der Hochdruckkammer erzeugt, wobei die Hochdruckkammer in Schwerkraftrichtung oberhalb der Niederdruckkammer angeordnet und mit einem oberen Deckel verschließbar ist,
dadurch gekennzeichnet,
daß die beiden Druckkammern (2,12) von einem verfahrbaren Jochrahmen (18) derart umschlossen sind, daß der Deckel (20) der Hochdruckkammer (12) und der Boden der Niederdruckkammer (2) sich bei aktiviertem Arbeitsdruck gegen Innenflächen (19,20) des Jochrahmens (18) abstützen.

2. Vorrichtung nach Anspruch 1,
gekennzeichnet durch
einen unteren Vorrichtungsrahmen (8), auf dem Halterungen (6) der Niederdruckkammer (2) federnd gelagert sind.

3. Vorrichtung nach Anspruch 1 oder 2,
dadurch gekennzeichnet,
daß auf dem Vorrichtungsrahmen (8) Fahrschienen (23) zum Verfahren des Jochrahmens (18) vorgesehen sind.

4. Vorrichtung nach einem der vorangehenden Ansprüche,
dadurch gekennzeichnet,
daß der Jochrahmen (18) aus einem Paket von Jochplatten (17) mit einer Innenausnehmung gebildet ist, die im wesentlichen der Außenkontur der Hoch- und Niederdruckkammer (2, 12) entspricht und diese in der Drucklage umschließt.

5. Vorrichtung nach einem der vorangehenden Ansprüche,
dadurch gekennzeichnet,
daß die Zylinderwand der Hochdruckkammer (12) mehrlagig ausgebildet ist mit einem Innenmantel (13) und einer Mehrzahl von diesen Innenmantel (13) umgebenden bandarmierten Ringscheiben (14).

6. Vorrichtung nach einem der vorangehenden Ansprüche,
gekennzeichnet durch
eine speicherprogrammierbare Steuerung (24) zur Regelung der Bedienungsabläufe sowie das Verfahren des Jochrahmens, der Deckelbetätigung, des Auf- und Abbaus des Arbeitsdruckes.

## Claims

1. Apparatus for producing a high pressure which is used for sterilisation purposes having a first high-pressure chamber as a working chamber and a second low-pressure chamber of larger diameter, wherein the two pressure chambers are acted upon by a tandem piston with piston surfaces of different sizes as a pressure booster and the tandem piston is activatable on its large surface by externally generated hydraulic pressure and thereby produces with its small surface the working pressure in the high-pressure chamber, wherein the high-pressure chamber is arranged above the low-pressure chamber in the direction of the force of gravity and is closable by an upper cover, characterised in that the two pressure chambers (2, 12) are surrounded by a displaceable yoke frame (18) in such a way that the cover (20) of the high-pressure chamber (12) and the bottom of the low-pressure chamber (2) are supported against inside surfaces (19, 20) of the yoke frame (18) when working pressure is activated.

2. Apparatus according to claim 1 characterised by a lower apparatus frame (8) on which holders (6) of the low-pressure chamber (2) are resiliently supported.

3. Apparatus according to claim 1 or claim 2 characterised in that rails (23) for displacement of the yoke frame (18) are provided on the apparatus frame (8).

4. Apparatus according to one of the preceding claims characterised in that the yoke frame (18) is formed from a pack of yoke plates (17) with an internal opening which substantially corresponds to the external contour of the high-pressure and low-pressure chambers (2, 12) and surrounds same in the pressure position.

5. Apparatus according to one of the preceding claims characterised in that the cylinder wall of the high-pressure chamber (12) is of a multi-layer configuration having an internal casing (13) and a plurality of band-reinforced annular discs (14) surrounding said internal casing (13).

6. Apparatus according to one of the preceding claims characterised by a memory-programmable control (24) for regulating the operating procedures and displacement of the yoke frame, cover actuation and building up and reducing the working pressure.

## Revendications

1. Dispositif pour la production de haute pression destiné à la stérilisation avec une première chambre haute pression en tant que chambre de travail et une seconde chambre basse pression de plus grand diamètre, les deux chambres de pression étant alimentées par un piston tandem avec des surfaces de piston de grosseurs différentes en tant que dispositif multiplicateur de pression et le piston tandem pouvant être activé sur sa grande surface par une pression hydraulique produite extérieurement et ainsi par sa petite surface produit la pression de travail dans la chambre haute pression, la chambre haute pression étant disposée dans la direction de la force centrifuge au-dessus de la chambre basse pression et pouvant être obturée par un couvercle supérieur,
caractérisé en ce que
les deux chambres de pression (2,12) sont entourées par un châssis portique (18) mobile de telle sorte que le couvercle (20) de la chambre haute pression (12) et le plancher de la chambre basse pression (2) s'appuient lors d'une pression de travail activé contre les surfaces internes (19, 20) du châssis portique (18).

2. Dispositif selon la revendication 1, caractérisé par un châssis de dispositif inférieur (8) sur lequel sont montées de façon élastique des fixations (6) de la chambre basse pression (2).

3. Dispositif selon la revendication 1 ou 2, caractérisé en ce que sur le châssis du dispositif (8) sont prévus des rails de déplacement (23) pour le déplacement du châssis portique (18).

4. Dispositif selon l'une des revendications précédentes, caractérisé en ce que le châssis portique (18) est constitué par un paquet de plaques portique (17) avec un évidement interne qui pour l'essentiel correspond aux contours externes des chambres haute et basse pressions (2,12) et renferme celles-ci dans la position de pression.

5. Dispositif selon l'une des revendications précédentes caractérisé en ce que la paroi cylindrique de la chambre haute pression (12) se présente en plusieurs couches avec une enveloppe interne (13) et une pluralité de disques annulaires (14) en armature feuillard entourant l'enveloppe interne (13).

6. Dispositif selon l'une des revendications précédentes, caractérisé par une commande à mémoire programmable (24) pour réguler les séquences d'actionnement ainsi que le déplacement du châssis portique, de l'actionnement du couvercle, de l'augmentation et de la réduction de la pression de travail.
